# EUROPEAN PATENT APPLICATION

(11) **EP 2 093 817 A1**
(43) Date of publication of application: **26.08.2009**
(21) Application number: 07831411.9
(22) Date of filing: 08.11.2007
(51) Int. Cl.: H01L 51/50, C09K 11/06, H05B 33/10

(54) **ORGANIC EL MATERIAL-CONTAINING SOLUTION, METHOD FOR FORMING THIN FILM OF ORGANIC EL MATERIAL, THIN FILM OF ORGANIC EL MATERIAL, AND ORGANIC EL DEVICE**

(30) Priority: 09.11.2006 JP 2006304628
(71) Applicant: Idemitsu Kosan Co., Ltd., Chiyoda-ku Tokyo 100-8321 (JP)
(72) Inventor: INOUE, Tetsuya, Sodegaura-shi Chiba 299-0293 (JP); IKEDA, Kiyoshi, Sodegaura-shi Chiba 299-0293 (JP); ITO, Mitsunori, Sodegaura-shi Chiba 299-0293 (JP); HOSOKAWA, Chishio, Sodegaura-shi Chiba 299-0293 (JP)
(74) Representative: Gille Hrabal Struck Neidlein Prop Roos
(86) International application number: PCT/JP2007/071680
(87) International publication number: WO 2008/056723

(57) **Abstract**

An organic EL material-containing solution contains an organic EL material and a solvent. The organic EL material contains a host and a dopant, the host being a naphthacene compound having a solubility in the solvent of 0.5 wt% or higher. The solvent is selected from the group consisting of an aromatic solvent, a halogen type solvent and an ether type solvent.

## Description

### TECHNICAL FIELD

The present invention relates to an organic EL material-containing solution, a method for forming a thin film of an organic EL material, the thin film of the organic EL material and an organic EL device. For example, the present invention relates to an organic EL material-containing solution which is used in forming an organic thin film that forms the organic EL device by a coating method.

### BACKGROUND ART

An organic EL (Electro-Luminescence) device is a self-luminescent device that is realized by utilizing a principle in which a fluorescent material emits light by recombination energy of a hole injected from an anode and an electron injected from a cathode by applying an electric field.

After a low-voltage drive organic EL device using a laminate device was reported by C. W. Tang et al of Eastman Kodak Company, organic EL devices formed from an organic material as a constituent have been eagerly studied.

Tang et al employ a lamination structure using a tris(8-quinolinol)aluminum for an emitting layer and a triphenyldiamine derivative for a hole transporting layer. Advantages of the lamination structure includes a capability of enhancing an injection efficiency of the hole into the emitting layer, a capability of blocking the electron injected by the cathode to enhance generation efficiency of exciton that is generated by recombination and a capability of trapping in the exciton generated in the emitting layer. As the structure of the organic EL device, a two-layer structure having the hole transporting (injecting) layer and an electron transporting emitting layer and a three-layer structure having the hole transporting (injecting) layer, the emitting layer and the electron transporting (injection) layer are well known. With such a laminate device, a variety of devisal has been made for structures and formation methods of the device in order to enhance the recombination efficiency of the injected hole and electron.

As luminescent materials used for the organic EL device, a chelate complex such as a tris(8-quinolinol)aluminum complex, a coumarin complex, a tetraphenylbutadiene derivative, a bisstyrylarylene derivative, an oxadiazole derivative are known. These luminescent materials have been reported to be capable of providing luminescence in a visible range from blue to red, and therefore it is expected to realize color display devices by using the materials (see, for instance, Patent Document 1 and Patent Document 2). However its luminous efficiency and lifecycle have not attained to a practically applicable level. Further, three primary colors (blue, green and red) are required for realizing a full-color display, and a red-color device having a high efficiency is especially required.

Although polymer materials and low-molecular materials are known as organic EL materials, development of low-molecular organic EL materials have been advanced due to its simple synthetic pathway and capability of high degree of purification. Among those developed low-molecular organic EL materials, organic EL materials having excellent efficiency, lifecycle and color purity have been proposed and put to practical application.
Vacuum deposition is employed for forming thin films using the low-molecular EL organic material, where the low-molecular EL organic material is sublimated with good thermal stability to vapor-deposit the thin films on a substrate, thereby obtaining a high-quality organic EL device.

However, the vapor deposition requires equipment capable of producing high vacuum and complicated manufacturing process.
Meanwhile, organic electroluminescence device using, as the luminescent material, polymer such as poly(p-phenylene vinylene) (PPV) and poly(2-methoxy-5-(2'-ethyl-hexyloxy)-1,4-phenylene vinylene) have been introduced (Non Patent Document 1: Nature, vol. 347, page 539, 1990 and Appl. Phys. Lett. vol. 58, page 1982, 1991).
In addition, a soluble PPV containing a functional group capable of enhancing solubility in an organic solvent has been developed. Due to the development, the emitting layer can be formed by wet film formation methods such as a spin coating and an ink jet printing using a solution containing a PPV derivative, thereby easily obtaining the device. The organic electroluminescence device using the PPV or the derivative thereof as the material of the emitting layer produces luminescence from green to orange.

Most of currently known luminescent low-molecular materials have poor solubility, which are deposited by vacuum deposition to form the emitting layer. However, the vacuum deposition has many disadvantages, i.e., requiring a complicated process and a large vapor-deposition device. Due to the disadvantages, it has been requested that film formation be performed easily by the wet film formation method even when the low-molecular compound is used. The luminescent low-molecular compound can be easily manufactured with a synthetic rout shorter than the PPV and can be purified with a high purity by a known technology such as column chromatography, which are advantageous. With the background, although attempts have been made to use a soluble low-molecular compound, crystallization occurs after the film formation by the wet film formation method and generates a pin hole in the film. Therefore, the soluble low-molecular compound cannot be used alone, and needs to be dispersed in a binder resin or the like when used for the film formation. However, the binder resin is electrically inactive, and therefore the binder resin might degrade luminescence property. As described above, it has been requested that the soluble luminescent compound be deposited by the wet film formation method to obtain a high quality emitting layer and that the device produced from the soluble emitting compound has a high luminous efficiency.

Patent Document 1: JP-A-H8-239655
Patent Document 2: JP-A-H7-138561
Non Patent Document 1: Nature, vol. 347, page 539, 1990 and Appl. Phys. Lett. vol. 58, page 1982, 1991

### DISCLOSURE OF THE INVENTION

### PROBLEMS TO BE SOLVED BY THE INVENTION

When the low-molecular organic EL material is deposited by a coating method, the low-molecular organic EL material needs to be dissolved in an organic solvent.
However, a material having a good red luminescent property which is selected in view of the luminous efficiency, lifecycle, chromaticity and the like is difficult to be dissolved in the organic solvent.
For example, JP-A-2002-008867 discloses a rubrene and a naphthacene compound as a host. However, since these substances have a poor solubility, it is difficult to control the thickness of a film or to form a uniform film in forming the film by coating.

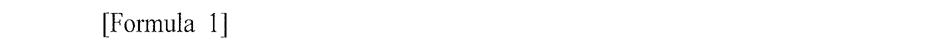

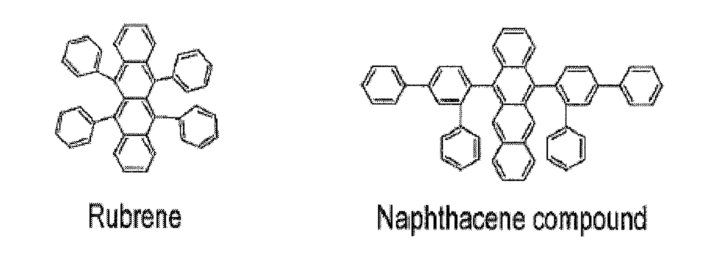

With the problems described above, the films cannot be formed easily and at low cost from the low-molecular organic EL material that has excellent luminous efficiency, lifecycle and color purity by the coating method, which is a severe obstacle in full-scale practical application of the organic EL materials.

An object of the present invention is to provide an organic EL material-containing solution that is free from the problems described above and therefore can be applied to a coating method.
Another object of the present invention is to provide a method for forming a thin film of an organic EL material, the thin film of the organic EL material and an organic EL device.

### MEANS FOR SOLVING THE PROBLEMS

An organic EL material-containing solution according to an aspect of the present invention contains an organic EL material and a solvent, in which the organic EL material contains a host and a dopant, the solvent contains a solvent selected from the group consisting of an aromatic solvent, a halogen type solvent and an ether type solvent, and the host is shown by General Formula (1) below, the host having a solubility in the solvent of 0.5 wt% or higher.

A film can be formed by a coating method using the organic EL material-containing solution as described above.
Meanwhile, typical organic EL materials do not usually have sufficient solubility in solvents.
Especially, an emitting layer (e.g., 30 nm to 100 nm) formed from a host and dopant needs to contain the host as a main part (e.g., 80% or more), and therefore low solubility of the host hinders the emitting layer from being formed in a predetermined thickness.
In this regard, according to the aspect of the invention, since the host constituting a main part of the emitting layer has a solubility of 0.5 wt% or higher, the emitting layer can be formed with a sufficient thickness.

Note that the solvent is at least one type selected from the group consisting of the aromatic solvent, the halogen type solvent and the ether type solvent, and two or more of them may be used in combination.

According to the aspect of the invention, the host is preferably shown by Formula (1) below.

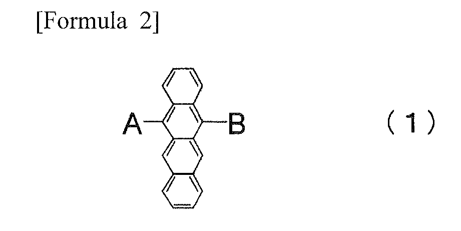

In Formula (1) above, A and B each representing a substituted or unsubstituted aromatic group having 6 to 20 carbon atoms or a substituted or unsubstituted fused aromatic group having 10 to 20 carbon atoms, where A and B may be the same or different from each other. At least one of A and B has a structure shown by General Formula (2) below.

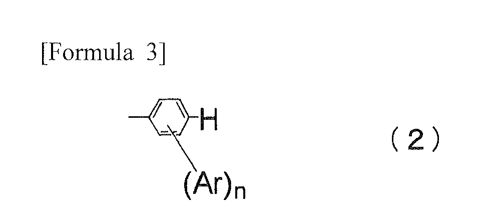

In the formula above, Ar represents a substituted or unsubstituted aromatic group having 6 to 20 carbon atoms or a substituted or unsubstituted fused aromatic group having 10 to 20 carbon atoms.
n represents an integer of 0 to 4.

With the structure described above, the solubility in the solvent can be a predetermined value or higher.
When an aromatic group is bonded as a substituent group to a naphthacene skeleton in a para position, the solubility becomes low. For example, a compound shown below has a solubility of 0.1 wt% or lower, which is extremely low.

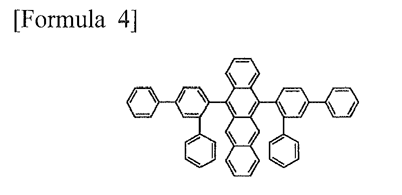

In this regard, according to the aspect of the invention, since the structure of Formula (2) above is provided as the substituent to the naphthacene skeleton, the solubility in the solvent can be a predetermined value or higher. With the structure, a compound exhibiting an excellent performance as an organic EL material and having a high solubility can be selected, thereby realizing the organic EL material-containing solution suitable for a film forming process by the coating method.

In Formula (2) above, n represents 0 to 4, where inclusion of 0 as n means that the compound shown by Formula (1) above does not contain two substituent groups in a para positions.

According to the aspect of the invention, in Formula (2) above, n preferably represents an integer of 0 to 2.

According to the aspect of the invention, in Formula (2) above, Ar preferably represents the substituted or unsubstituted aromatic group having 6 to 20 carbon atoms.

According to the aspect of the present invention, it is preferable that the solvent is selected from the group consisting of the aromatic solvent, the halogen type solvent and the ether type solvent, the host is shown by Formula (1) above, the host having a solubility in the solvent of 2 wt% or higher, and a viscosity control reagent is added to the solvent, the viscosity control reagent being selected from the group consisting of an alcohol type solution, a ketone type solution, a paraffin type solution and an alkyl-substituted aromatic solution having 4 or more carbon atoms,
(in Formula (2) above, Ar being selected from the group consisting of phenyl, naphthyl and biphenyl).

In the solution having the composition as described above, a film can be formed by the coating method by dissolving the organic EL material (the host and the dopant) in the solvent.
Here, the organic EL material-containing solution for the film formation by the coating method requires to have a viscosity of a predetermined level or higher as well as containing the organic EL material by a predetermined amount or more.
For example, when a film is formed by methods such as a spin coating, an ink jet printing and a nozzle printing, a viscosity as the solution requires to be several cPs or higher.
Since low-molecular organic EL materials typically have poor solubility and do not exhibit high viscosity even when being dissolved, it is difficult to select a solvent that can dissolve the low-molecular organic EL materials while achieving a sufficient viscosity. Generally, solvents suitable for viscosity control are poor solvents.
However, according to the aspect of the invention, by separately selecting a solvent for dissolving the low-molecular organic EL material and a viscosity control reagent for controlling the viscosity, both the sufficient solubility and viscosity can be achieved at the same time.
In the aspect of the present invention, the host requires to have a solubility of not a minimum required level as the solubility of the host (e.g., 0.5 wt%) but a sufficiently high level (2 wt%) and requires to have a structure that can realize such a high solubility. To satisfy such requirements, compounds having a solubility of a predetermined level or higher have been selected from compounds soluble in solvents. As described above, since the host has a sufficiently high solubility in the solvent, the viscosity control reagent for the viscosity control can be additionally added as a thickener. Accordingly, the organic EL material-containing solution having a viscosity of 1 cP or higher and an amount of dissolution of 0.5 wt% or higher can be obtained.
Since the host has the sufficiently high solubility, a poor solvent can be selected as the viscosity control reagent, thereby facilitating the viscosity control and selection of the solvent.

Note that the viscosity control reagent is at least one type selected from the group consisting of the alcohol type solution, the ketone type solution, the paraffin type solution, an alkyl-substituted aromatic solution and an alkyl-substituted aromatic solution having 4 or more carbon atoms, and two or more of them may be used in combination.

The "alkyl-substituted aromatic solution having 4 or more carbon atoms" is an alkyl-substituted group that is aromatic and has 4 or more carbon atoms.
Although the upper limit of the number of carbon atoms of the alkyl-substituted group is not particularly set, the upper limit may be, for instance, approximately 50.

According to the aspect of the invention, in Formula (2) above, Ar preferably represents a substituted or unsubstituted phenyl group.

By selecting the phenyl group as the substituent group in Formula (2) above, the solubility can be increased and the performance as the organic EL material can be enhanced.

According to the aspect of the invention, the solvent is the aromatic solvent, and the viscosity control reagent is preferably selected from the group consisting of the alcohol type solution and the alkyl-substituted aromatic solution having 4 or more carbon atoms.

Here, when the alcohol type solution is selected as the viscosity control reagent, storage of the resulting solution requires close attention since the alcohol type solution easily absorbs moisture. However, by selecting the alkyl-substituted aromatic solution having 4 or more carbon atoms as the viscosity control reagent, which is hydrophobic, the storage of the resulting solution can be facilitated.
In addition, the alkyl-substituted aromatic solution having 4 or more carbon atoms is capable of controlling the viscosity by changing the structure of the alkyl group (for instance, by prolonging an alkyl chain).
On the other hand, the alcohol type solution is preferable in preparing a solution that is suitable for a film forming process requiring high solution viscosity (e.g., ink jet printing) due to its high viscosity.

A type or an adding amount of the viscosity control reagent can be properly selected in accordance with the viscosity required for various types of film forming processes.

According to the aspect of the invention, the dopant is preferably an indenoperylene derivative shown by Formula (3) below.

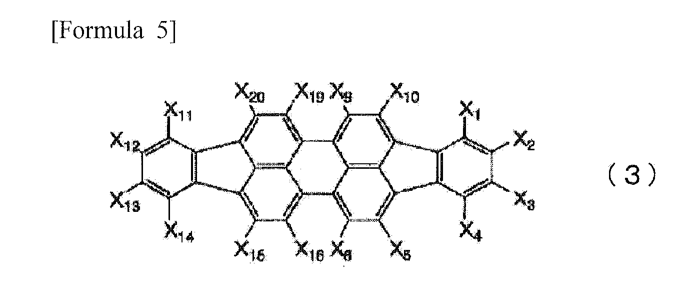

(In Formula (3), X₁ to X₆, X₉, X₁₀, X₁₁ to X₁₆, X₁₉ and X₂₀ each represent a hydrogen, a halogen, an alkyl group, an alkoxy group, an alkylthio group, an alkenyl group, an alkenyloxy group, an alkenylthio group, an aromatic ring-containing alkyl group, an aromatic ring-containing alkyloxy group, an aromatic ring-containing alkylthio group, an aromatic ring group, an aromatic heterocyclic group , an aryloxy group, an arylthio group, an arylalkenyl group, an alkenylaryl group, an amino group, a carbazolyl group, a cyano group, a hydroxyl group, -COOR^{1'} (R^{1'} representing a hydrogen, an alkyl group, an alkenyl group, an aromatic ring-containing alkyl group or an aromatic ring group), -COR^{2'} (R^{2'} representing a hydrogen, an alkyl group, an alkenyl group, an aromatic ring-containing alkyl group, an aromatic ring group or an amino group) or -OCOR^{3'} (R^{3'} representing an alkyl group, an alkenyl group, an aromatic ring-containing alkyl group or an aromatic ring group).
Adjacent groups of X₁ to X₆, X₉, X₁₀, X₁₁ to X₁₆, X₁₉ and X₂₀ may be bonded to each other or bonded to a substituted carbon atom to form a ring. At least one of X₁ to X₆, X₉, X₁₀, X₁₁ to X₁₆, X₁₉ and X₂₀ is not the hydrogen.)

According to the aspect of the invention, the dopant is preferably an indenoperylene derivative shown by Formula (4) below.

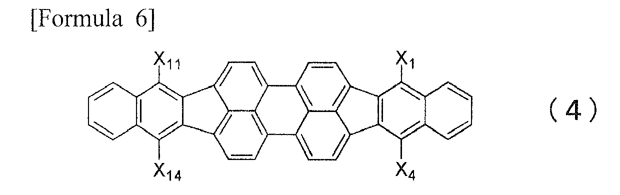

In Formula (4) above, X₁, X₄, X₁₁, X₁₄ are an aromatic ring group.
The aromatic ring group is preferably a phenyl group, an ortho-biphenyl group, a meta biphenyl group or a naphthyl group, and more preferably the phenyl group or the ortho-biphenyl group.

A method of forming thin film(s) of the organic EL material according to another aspect of the present invention includes: dropping the organic EL material-containing solution described above on a formation area; and forming film(s) of the organic EL material by evaporating the solvent in the organic EL material-containing solution dropped in the step of dropping.

A thin film of an organic EL material according to still another aspect of the present invention is formed by the method of forming thin film(s) of the organic EL material described above.

An organic EL device according to yet another aspect of the present invention includes the thin film of the organic EL material described above.

It should be noted that the organic EL material-containing solution of the present invention may be used by adding additives thereto such that the viscosity, boiling point and concentration are controlled to be suitable for a certain coating method, in addition to be used as it is as the solution for the coating.
For example, since the spin coating, the nozzle printing and the ink jet printing required different viscosities, viscosity control reagents may be appropriately added.

### BEST MODE FOR CARRYING OUT THE INVENTION

An embodiment of the present invention will be described below.

### (First Embodiment)

An organic EL material-containing solution of the present invention is prepared by dissolving an organic EL material in a solvent.
The organic EL material-containing solution contains a host and a dopant.

The host is a naphthacene compound shown below.

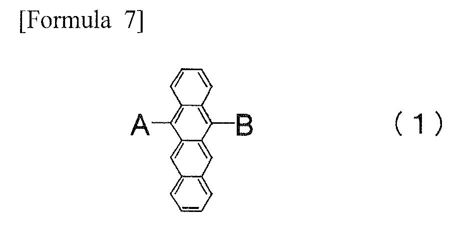

In Formula (1) above, A and B each represent a substituted or unsubstituted aromatic group having 6 to 20 carbon atoms or a substituted or unsubstituted fused aromatic group having 10 to 20 carbon atoms, where A and B may be the same or different from each other.
Herein, at least one of A and B has a structure shown by Formula (2) below.

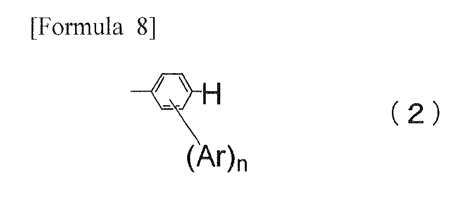

In the formula above, Ar represents a substituted or unsubstituted aromatic group having 6 to 20 carbon atoms or a substituted or unsubstituted fused aromatic group having 10 to 20 carbon atoms. n represents an integer of 0 to 4.

Here, examples of the substituent may include a hydrogen atom, a substituted or unsubstituted aromatic group having 6 to 50 carbon atoms forming the aromatic ring, a substituted or unsubstituted aromatic heterocyclic group having 5 to 50 atoms forming a ring, a substituted or unsubstituted alkyl group having 1 to 50 carbon atoms, a substituted or unsubstituted cycloalkyl group, a substituted or unsubstituted alkoxy group having 1 to 50 carbon atoms, a substituted or unsubstituted aralkyl group having 6 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 1 to 50 carbon atoms, a substituted or unsubstituted silyl group, a carboxyl group, a halogen atom, a cyano group, a nitro group and a hydroxyl group.

Specifically, there can be exemplified compounds shown below.

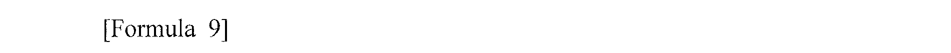

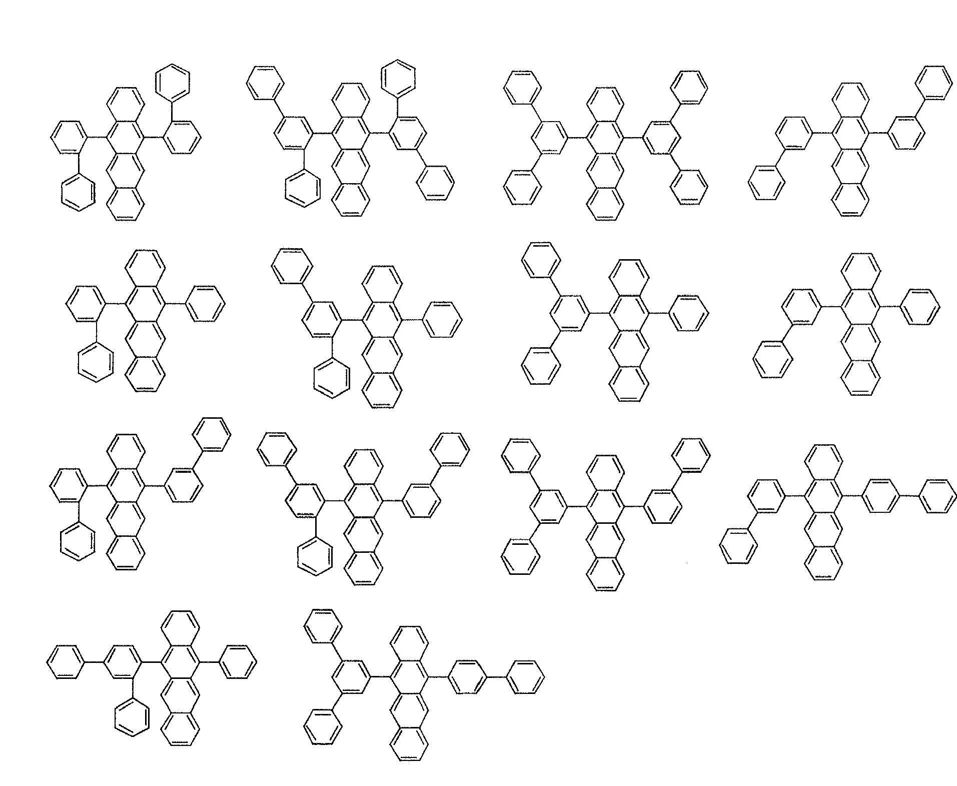

The dopant material is an indenoperylene derivative represented by General Formula below.

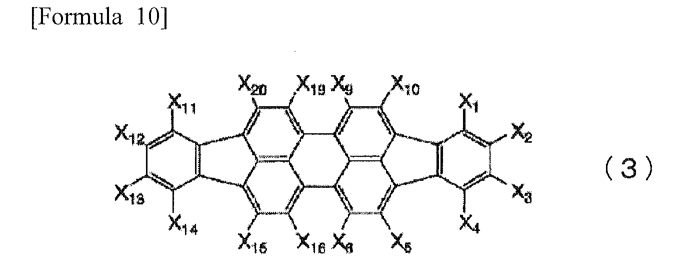

(In the formula above, X₁ to X₆, X₉, X₁₀, X₁₁ to X₁₆, X₁₉, and X₂₀ each represent a hydrogen, a halogen, an alkyl group, an alkoxy group, an alkylthio group, an alkenyl group, an alkenyloxy group, an alkenylthio group, an aromatic ring-containing alkyl group, an aromatic ring-containing alkyloxy group, an aromatic ring-containing alkylthio group, an aromatic ring group, an aromatic heterocyclic group , an aryloxy group, an arylthio group, an arylalkenyl group, an alkenylaryl group, an amino group, a carbazolyl group, a cyano group, a hydroxyl group, -COOR^{1'} (R^{1'} representing a hydrogen, an alkyl group, an alkenyl group, an aromatic ring-containing alkyl group or an aromatic ring group), -COR^{2'} (R^{2'} representing a hydrogen, an alkyl group, an alkenyl group, an aromatic ring-containing alkyl group, an aromatic ring group or an amino group) or -OCOR^{3'} (R^{3'} representing an alkyl group, an alkenyl group, an aromatic ring-containing alkyl group or an aromatic ring group).
Adjacent groups of X₁ to X₆, X₉, X₁₀, X₁₁ to X₁₆, X₁₉, and X₂₀ may be bonded to each other or bonded to a substituted carbon atom to form a ring. At least one of X₁ to X₆, X₉, X₁₀, X₁₁ to X₁₆, X₁₉, and X₂₀ is not the hydrogen.)

The dopant may be an indenoperylene derivative shown by Formula (4) below.

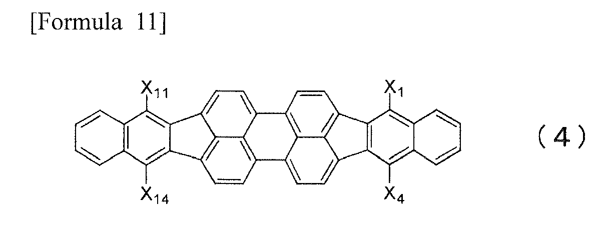

In formula (4) above, X_{1,} X₄, X₁₁, and X₁₄ are an aromatic ring group. The aromatic ring group is preferably selected from the group consisting of a phenyl group, an ortho-biphenyl group, a meta biphenyl group and a naphthyl group, and more preferably selected from the group consisting of the phenyl group and the ortho-biphenyl group.

The solvent is selected from the group consisting of an aromatic solvent, a halogen type solvent and an ether type solvent. The solvent is preferably the aromatic solvent.
The viscosity control reagent is selected from the group consisting of an alcohol type solution, a ketone type solution, a paraffin type solution and an alkyl-substituted aromatic solution having 4 or more carbon atoms.
The viscosity control reagent is the alcohol type solution or the alkyl-substituted aromatic solution having 4 or more carbon atoms.

The aromatic solvent may be exemplified by benzene, toluene, xylene, ethylbenzene, mesitylene and chlorobenzene.
A halogenated hydrocarbon solvent as the halogen type solvent may be exemplified by dichloromethane, dichloroethane, chloroform, carbon tetrachloride, tetrachloroethane, trichloroethane, chlorobenzene, dichlorobenzene and chlorotoluene.
The ether type solvent may be exemplified by dibutyl ether, tetrahydrofuran, dioxane and anisole.
The solvent may be, as the hydrocarbon solvent, hexane, octane, decane and the like.
The solvent may be, as an ester solvent, ethyl acetate, butyl acetate, amyl acetate and the like.
The alcohol type solution may be exemplified by methanol, ethanol, propanol, butanol, pentanol, hexanol, octanol, cyclohexanol, methyl cellosolve, ethylcellosolve, ethylene glycol and benzyl alcohol. The alcohols described above may be straight chained or branched.
The alkyl-substituted aromatic solution having 4 or more carbon atoms may be exemplified by an alkyl benzene derivative having 4 or more carbon atoms, examples of which include a straight-chained or branched butylbenzene, dodecylbenzene, tetralin and cyclohexylbenzene. The alkyl-substituted group described above may be straight chained or branched.

Now, examples and comparisons of the present invention will be described.

### (Solubility Evaluation)

A description about solubility evaluation will be given below.
As the solubility evaluation, solubilities of hosts were evaluated.

### (Examples 1 and 2, Comparisons 1 to 3)

A hundred mg of a compound was placed in a sample pot, to which toluene was added until the compound was dissolved.
The evaluation was conducted on Compounds H1 to H5 below.
From an addition amount of the toluene, solubility in the toluene was obtained.
The result is shown in Table 1.

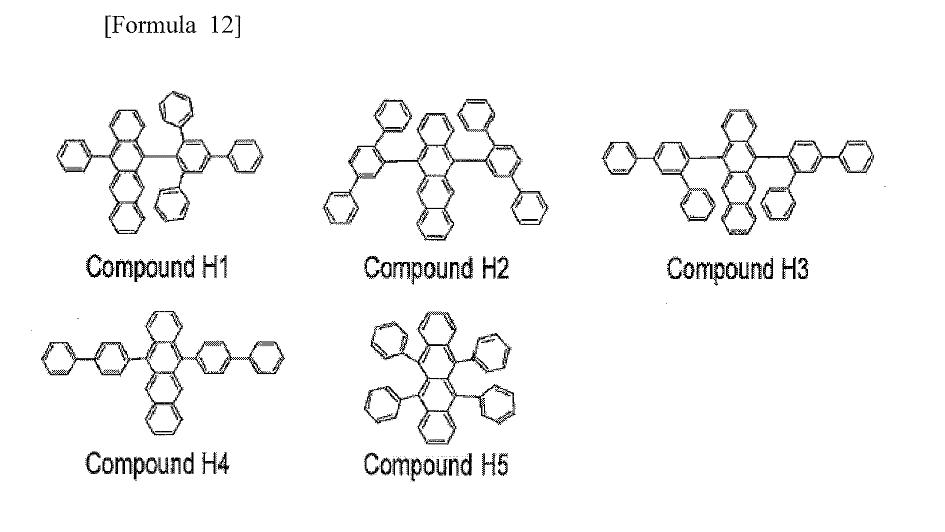

Solubility of Compound in Toluene

**[Table 1]**

| | Example 1 | Example 2 | Comparison 1 | Comparison 2 | Comparison 3 |
|---|---|---|---|---|---|
| Compound | Compound H1 | Compound H2 | Compound H3 | Compound H4 | Compound H5 |
| Solubility(%) | 0.7 | 0.3 | 0.1 or lower | 0.1 or lower | 0.1 or lower |

From the results of Examples 1 and 2 and Comparisons 1 to 3, Compounds H3 and H4 having substitutions in a para position is two showed extremely low solubility in the toluene, and thus verified to be difficult to form a film by a wet process.
Further, Compound H5 having no substitution in a para position showed solubility lower than those of Compounds H1 and H2.

Next, inks as organic EL material-containing solutions were prepared in Examples 3 to 5 and Comparisons 4 and 5.

### (Example 3)

Compound H2 (100 mg) and Compound A (5 mg) were mixed in a sample pot, to which toluene (1 g) was added.
Then, 1 g of 1-octyl alcohol was added and the mixture was agitated.
No insoluble matter was observed at this time and the viscosity was 1.6 cP.

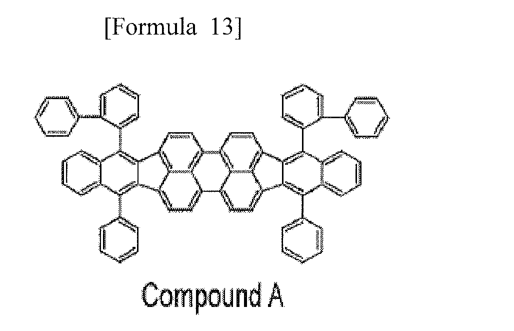

### (Example 4)

An ink was prepared similarly to Example 3 except that 1 g of 2-ethylhexyl alcohol was used instead of 1g of the 1-octyl alcohol. No insoluble matter was observed at this time and the viscosity was 1.5 cP.

### (Example 5)

An ink was prepared similarly to Example 3 except that 1 g of benzyl alcohol was used instead of 1g of the 1-octyl alcohol. No insoluble matter was observed at this time and the viscosity was 1.5 cP. No solid precipitate was observed for more than 1 week in this solution.

### (Comparison 4)

An ink was prepared similarly to Example 3 except that 1 g of 2-toluene was used instead of 1g of the 1-octyl alcohol. No insoluble matter was observed at this time and the viscosity was 0.65 cP.

### (Comparison 5)

Compound H5 (100 mg) and Compound A (5 mg) were mixed in a sample pot, to which toluene (1 g) was added. Then, 1 g of 1-octyl alcohol was added and the mixture was agitated. As a result, a solid precipitate was observed.

As described above, it was verified that alcohol solutions for solution viscosity control could be added by selecting naphthacene compounds having high solubility.

### (Organic EL Device)

Next, an organic EL device will be described.

### (Arrangement of Organic EL Device)

A description about an arrangement of the organic EL device will be given.

### [1] Arrangement of Organic EL Device

Typical arrangement of the organic EL device may be exemplified by the following arrangements.
(a) anode/emitting layer/cathode
(b) anode/hole injecting layer/emitting layer/cathode
(c) anode/emitting layer/electron injecting layer/cathode
(d) anode/hole injecting layer/emitting layer/electron injecting layer/cathode
(e) anode/organic semiconductor layer/emitting layer/cathode
(f) anode/organic semiconductor layer/electron blocking layer/emitting layer/cathode
(g) anode/organic semiconductor layer/emitting layer/adhesion improving layer/cathode
(h) anode/hole injecting layer/hole transporting layer/emitting layer/electron injecting layer/cathode
(i) anode/insulating layer/emitting layer/insulating layer/cathode
(j) anode/inorganic semiconductor layer/insulating layer/emitting layer/insulating layer/cathode
(k) anode/organic semiconductor layer/insulating layer/emitting layer/insulating layer/cathode
(l) anode/insulating layer/hole injecting layer/hole transporting layer/emitting layer/insulating layer/cathode
(m) anode/insulating layer/hole injecting layer/hole transporting layer/emitting layer/electron injecting layer/cathode

Among these, the arrangement (h) is usually preferable.

### [2] Light-Transmissive Substrate

The organic EL device is formed on a light-transmissive substrate. The light-transmissive substrate used herein is a substrate supporting the organic EL device, which is preferably a flat substrate having a transmittance of 50% or higher for a light in the visible range of 400 to 700 nm.
Specifically, a glass plate, a polymer plate and the like may be employed.
Particularly, the glass plate may include a soda-lime glass, a barium/strontium-containing glass, a lead glass, an aluminosilicate glass, a borosilicate glass, a barium borosilicate glass and a quartz.
The polymer plate may include a polycarbonate, an acryl, a polyethylene terephthalate, a polyether sulfide and a polysulfone.

### [3] Anode

The anode of the organic EL device injects a hole in the hole transporting layer and the emitting layer, so that it is efficient that the anode has a work function of 4.5 eV or higher. Concrete examples of an anode material may include indium-tin oxide (ITO), tin oxide (NESA), indium zinc oxide (IZO), gold, silver, platinum and copper. The anode with smaller work function is more preferable in order to inject an electron to the electron injecting layer and the emitting layer.
The anode may be made by forming a thin film from these electrode substances through methods such as vapor deposition and sputtering.
When emission from the emitting layer is taken out from the anode, the anode preferably has a transmittance of higher than 10% for the emission. The sheet resistance of the anode is preferably several hundreds Ω/ square or lower. The thickness of the anode is typically in the range from 10 nm to 1 µm, and preferably in the range from 10 nm to 200 nm, though it depends on the material of the anode.

### [4] Emitting Layer

The emitting layer of the organic EL device has functions below:
Injecting function: a function for allowing the hole to be injected thereto by the anode or the hole injecting layer, or for allowing the electron to be injected thereto by the cathode or the electron injecting layer when an electrical field is applied;
Transporting function: a function for transporting injected electric charges (the electron and the hole) by the force of the electrical field; and Emitting function; a function for providing a condition for recombination of the electron and the hole to generate emission.
Herein, although there may be a difference in degrees of easiness of receiving the injected hole and that of the injected electron and a difference in transporting capabilities represented by mobilities of the hole and the electron, the emitting layer preferably transports one of the electric charges.
Conventional methods such as vapor deposition, spin coating and an LB method may be employed as a method for forming the emitting layer.
The emitting layer is particularly preferably a molecular deposition film.
Here, the molecular deposition film is a thin film that is formed by depositing a material compound in the gas phase or a film formed by solidifying a material compound in a solution state or the liquid phase. The molecular deposition film can be typically distinguished from a thin film formed by the LB method (molecular built-up film) by differences in aggregation structures and higher order structures and differences in resulting functions.
In addition, as disclosed in JP-A-57-51781, the emitting layer can be formed by preparing a solution by dissolving a binder such as a resin and the material compound in a solvent and forming a thin film from the solution by spin coating or the like.
The thickness of the emitting layer is preferably in the range from 5 nm to 50 nm, more preferably in the range from 7 nm to 50 nm and most preferably in the range 10 nm to 50 nm. The thickness below 5 nm may cause difficulty in forming the emitting layer and in controlling chromaticity, while the thickness above 50 nm may raise driving voltage.

### [5] Hole Injecting/Transporting Layers (Hole Transporting Zone)

The hole injecting/transporting layer helps injection of the hole to the emitting layer and transport the hole to an emitting region, in which the hole mobility is large and the energy of ionization is typically small (5.5 eV or smaller). A material of the hole injecting/transporting layer is preferably those transporting the hole to the emitting layer with a low field intensity, and more preferably those transporting the hole with the hole mobility of, for example, 10⁴ to 10⁶ V/cm or at least 10⁻⁴ cm²/V·sec when the electrical field is applied.

Concrete examples of the material may include a triazole derivative (see, for instance, the specification of US Patent No. 3,112,197), an oxadiazole derivative (see, for instance, the specification of US Patent No. 3,189,447), an imidazole derivative (see, for instance, the publication of JP-B-37-16096), a polyarylalkane derivative (see, for instance, the specifications of US Patent No. 3,615,402, No.3,820,989 and No. 3,542,544 and the publications of JP-B-45-555, JP-B-51-10983, JP-A-51-93224, JP-A-55-17105, JP-A-56-4148, JP-A-55-108667, JP-A-55-156953, and JP-A-56-36656), a pyrazoline derivative and a pyrazolone derivative (see, for instance, the specifications of US Patent No. 3,180,729 and No. 4,278,746 and the publications of JP-A-55-88064, JP-A-55-88065, JP-49-105537, JP-A-55-51086, JP-A-56-80051, JP-A-56-88141, JP-A-57-45545, JP-A-54-112637 and JP-A-55-74546, a phenylenediamine derivative (see, for instance, the specification of US Patent No. 3,615,404 and the publications of JP-B-51-10105, JP-B-46-3712, JP-B-47-25336, and JP-A-54-119925), an arylamine derivative (see, for instance, the specifications of US Patent No. 3,567,450, No. 3,240,597, No. 3,658,520, No. 4,232,103, No. 4,175,961 and No. 4,012,376 and the publications of JP-B-49-35702, JP-B-39-27577, JP-A-55-144250, JP-A-56-119132 and JP-A-56-22437 and the specification of West Germany Patent No. 1,110,518), an amino-substituted chalcone derivative (see, for instance, the specification of US Patent No. 3,526,501), an oxazole derivative (disclosed in, for instance, the specification of US Patent No. 3, 257,203), a styrylanthracene derivative (see, for instance, the publication of JP-A-56-46234), a fluorenone derivative (see, for instance, the publication of JP-A-54-110837), a hydrazone derivative (see, for instance, the specification of US Patent No. 3,717,462 and the publications of JP-A-54-59143, JP-A-55-52063, JP-A-55-52064, JP-A-55-46760, JP-A-57-11350, JP-A-57-148749 and JP-A-2-311591), a stilbene derivative (see, for instance, the publications of JP-A-61-210363, JP-A-61-228451, JP-A-61-14642, JP-A-61-72255, JP-A-62-47646, JP-A-62-36674, JP-A-62-10652, JP-A-62-30255, JP-A-60-93455, JP-A-60-94462, JP-A-60-174749 and JP-A-60-175052), a silazane derivative (see the specification of US Patent No. 4,950,950), a polysilane type (see the publication of JP-A-2-204996), an aniline-type copolymer (see the publication of JP-A-2-282263), and a conductive polymer oligomer (thiophene oligomer) .

Although the substances listed above can be used as the materials of the hole injecting/transporting layers, it is preferable to use a porphyrin compound (disclosed in, for instance, the publication of JP-A-63-295695), an aromatic tertiary amine compound and a styrylamine compound (see, for instance, the publication of US Patent No. 4,127,412 and the publications of JP-A-53-27033, JP-A-54-58445, JP-A-55-79450, JP-A-55-144250, JP-A-56-119132, JP-A-61-29558, JP-A-61-98353 and JP-A-63-295695), and among these, the aromatic tertiary amine compound is particularly preferable.
In addition, 4,4'-bis(N-(1-naphthyl)-N-phenylamino)biphenyl (hereinafter, abbreviated as NPD) having in the molecule two fused aromatic rings disclosed in US Patent No. 5,061,569, 4,4',4"-tris(N-3-methylphenyl-N-phenyl-amino)triphenylamine (hereinafter, abbreviated as MTDATA) in which three triphenylamine units disclosed in the publication of JP-A-4-30868 are bonded in a starbust form and the like may also be used.

In addition to the aromatic dimethylidine compound mentioned above as the material of the emitting layer, compounds such as p-type Si and p-type SiC can be used as the material of the hole injecting layer.

The hole injecting/transporting layer can be made by forming thin films from the compounds listed above by conventional methods such as a vacuum deposition, the spin coating, a casting method and the LB method.
The thickness of the hole injecting/transporting layer is not particularly limited, but typically in the range from 5 nm to 5 µm.

### [6] Electron Injecting/Transporting Layer (Electron Transporting Zone)

The electron injecting/transporting layer may further be laminated between the organic emitting layer and the cathode. The electron injecting/transporting layer helps injection of the electron to the emitting layer and has a high electron mobility.
It is known that, in the organic EL, since light emitted by the organic EL is reflected by an electrode (the cathode, in this case), light directly taken out from the anode and the light taken out after being reflected by the electrode interfere with each other. In order to efficiently utilize the interference, the thickness of the electron transporting layer is properly selected from the range of several nanometers to several micrometers. However, especially when the thickness of the layer is large, it is preferable that the electron mobility is at least 10⁻⁵ cm²/Vs or higher under the condition where the electrical field of 10⁴ to 10⁶ V/cm is applied to prevent voltage rise.
As a material used for the electron injecting/transporting layer, 8-hydroxyquinoline or a metal complex of its derivative is preferable. Concrete examples of the 8-hydroxyquinoline or the metal complex of its derivative may include metal chelate oxynoid compounds including a chelate of oxine (typically 8-quinolinol or 8-hydroxyquinoline). For example, Alq having Al as its central metal can be used for the electron injecting/transporting layer.

An oxadiazole derivative shown by the formula below is also preferable as a material for the electron injecting (transporting) layer.

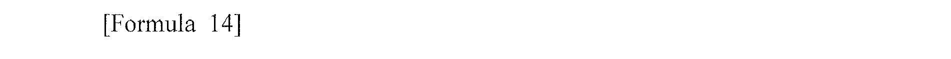

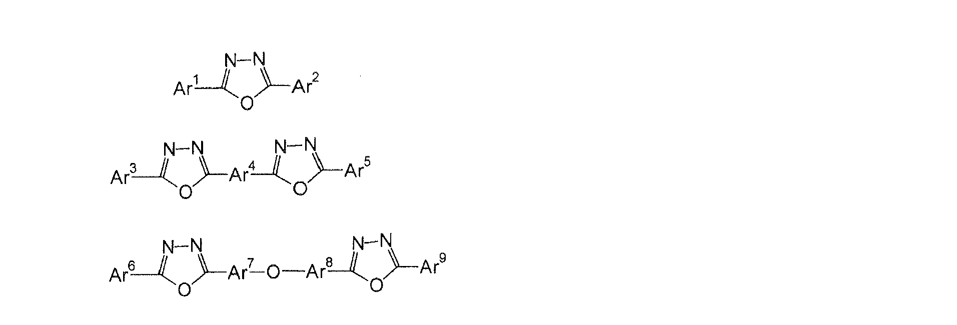

(In the formula, Ar¹ ,Ar² ,Ar³ ,Ar⁵ ,Ar⁶ and Ar⁹ each represent a substituted or unsubstituted aryl group, which may be the same or different from each other. Ar⁴,Ar⁷ and Ar⁸ each represent a substituted or unsubstituted arylene group, which may be the same or different from each other.

The aryl group may include a phenyl group, a biphenyl group, an anthranil group, a perylenyl group, and a pyrenyl group. The arylene group may include a phenylene group, a naphtylene group, a biphenylene group, an anthranylene group, a perylenylene group and a pyrenylene group. The substituent group may include an alkyl group having 1 to 10 carbon atoms, an alkoxy group having 1 to 10 carbon atoms and a cyano group. The electron transporting compounds are preferably those exhibiting good performance in forming a thin film.
Concrete examples of the electron transporting compounds may include substances below.

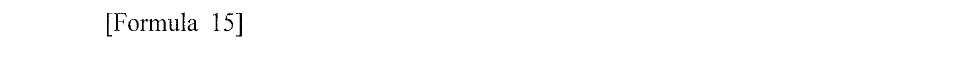

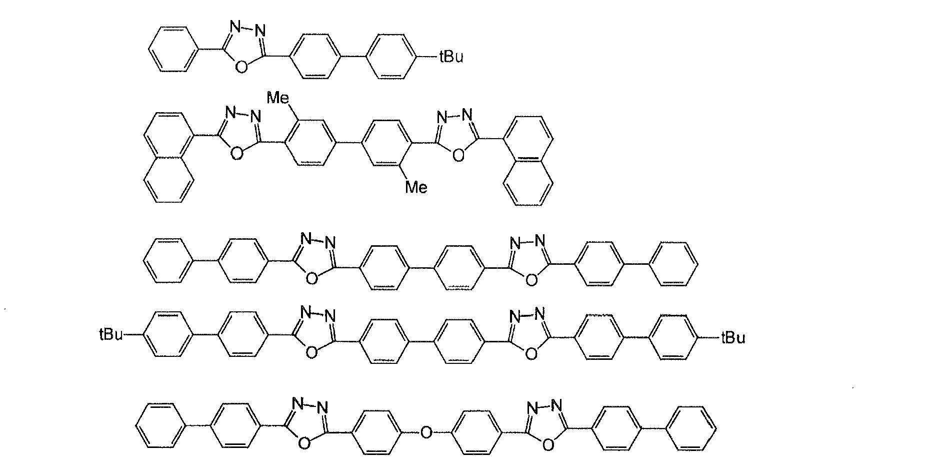

A nitrogen-containing heterocycle derivative shown by the formula below is also preferable as a material of the electron injecting (transporting) layer.

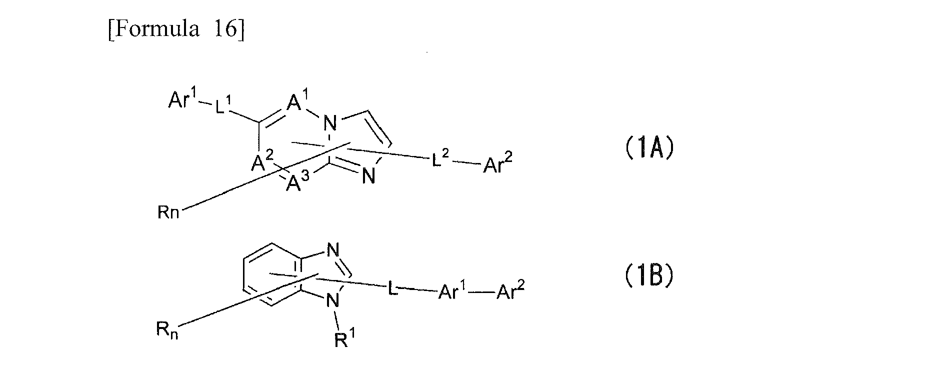

(In the formula, A¹ to A³ each represent a nitrogen atom or a carbon atom; R represents an aryl group having 6 to 60 carbon atoms which may have a substituent group, a heteroaryl group having 3 to 60 carbon atoms which may have a substituent group, an alkyl group having 1 to 20 carbon atoms, a haloalkyl group having 1 to 20 carbon atoms or an alkoxy group having 1 to 20 carbon atoms; and n represents an integer of 0 to 5, where the plurality of R may be the same or different from each other when n is an integer equal to or larger than two.
In addition, a plurality of adjacent R may be bonded to each other to form a substituted or unsubstituted carbocyclic aliphatic ring or a substituted or unsubstituted carbocyclic aromatic ring.
Ar¹ represents the aryl group having 6 to 60 carbon atoms which may have the substituent group or the heteroaryl group having 3 to 60 carbon atoms which may have the substituent group; and Ar² represents a hydrogen atom, the alkyl group having 1 to 20 carbon atoms, the haloalkyl group having 1 to 20 carbon atoms, the alkoxy group having 1 to 20 carbon atoms, the aryl group having 6 to 60 carbon atoms which may have the substituent group or the heteroaryl group having 3 to 60 carbon atoms which may have the substituent group, one of Ar¹ and Ar² being a fused ring group having 10 to 60 carbon atoms which may have a substituent group or a fused heterocyclic group having 3 to 60 carbon atoms which may have a substituent group.
L¹ and L² each represent a single bond, a fused ring having 6 to 60 carbon atoms which may have a substituent group, a fused heterocycle having 3 to 60 carbon atoms which may have a substituent group or a fluorenylene group which may have a substituent group.)

HAr-L¹-Ar¹-Ar² [Formula 17]

(In the formula, HAr represents a nitrogen-containing ring having 3 to 40 carbon atoms which may have a substituent group; L¹ represents a single-bonded arylene group having 6 to 60 carbon atoms which may have a substituent group, a heteroarylene group having 3 to 60 carbon atoms which may have a substituent group or a fluorenylene group which may have a substitute group; Ar¹ represents a divalent aromatic hydrocarbon group having 6 to 60 carbon atoms which may have a substituent group; and Ar² represents an aryl group having 6 to 60 carbon atoms which may have a substituent group, a heteroaryl group having 3 to 60 carbon atoms which may have a substituent group.)

A silacyclopentadiene derivative shown by the formula below is also preferable as a material of the electron injecting (transporting) layer.

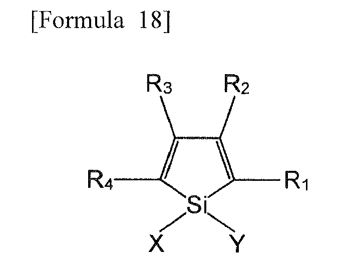

(In the formula, X and Y have a structure in which: X and Y each represent a saturated or unsaturated hydrocarbon group, having a carbon number of 1 to 6, an alkoxy group, an alkenyloxy group, an alkynyloxy group, a hydroxy group, a substituted or unsubstituted aryl group, a substituted or unsubstituted heterocycle; or X and Y are bonded to form a saturated or unsaturated ring. R₁ to R ₄ have a structure in which: each of R₁ to R₄ independently represents hydrogen, halogen, a substituted or unsubstituted alkyl group having a carbon number of 1 to 6, an alkoxy group, an aryloxy group, a perfluoroalkyl group, a perfluoroalkoxy group, an amino group, an alkylcarbonyl group, an arylcarbonyl group, an alkoxycarbonyl group, an aryloxycarbonyl group, an azo group, an alkylcarbonyloxy group, an arylcarbonyloxy group, an alkoxycarbonyloxy group, an aryloxycarbonyloxy group, a sulfinyl group, a sulfonyl group, a sulfanyl group, a silyl group, a carbamoyl group, an aryl group, a heterocyclo group, an alkenyl group, an alkynyl group, a nitro group, a formyl group, a nitroso group, a formyloxy group, an isocyano group, a cyanate group, an isocyanate group, a thiocyanate group, an isothiocyanate group or cyano group; or an adjacent set of R₁ to R₄ are fused to form a substituted or unsubstituted ring.)

A silacyclopentadiene derivative shown by the formula below is also preferable as a material of the electron injecting (transporting) layer.

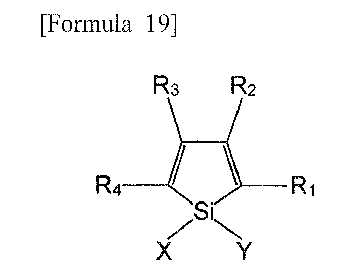

(In the formula, X and Y have a structure in which: X and Y each represent a saturated or unsaturated hydrocarbon group having 1 to 6 carbon atoms, an alkoxy group, an alkenyloxy group, an alkynyloxy group, a substituted or unsubstituted aryl group, a substituted or unsubstituted heterocycle; or X and Y are bonded to form a saturated or unsaturated ring. R₁ to R₄ have a structure in which: R₁ to R₄ each represent hydrogen, halogen, a substituted or unsubstituted alkyl group having 1 to 6 carbon atoms, an alkoxy group, an aryloxy group, a perfluoroalkyl group, a perfluoroalkoxy group, an amino group, an alkylcarbonyl group, an arylcarbonyl group, an alkoxycarbonyl group, an aryloxycarbonyl group, an azo group, an alkylcarbonyloxy group, an arylcarbonyloxy group, an alkoxycarbonyloxy group, an aryloxycarbonyloxy group, a sulfinyl group, a sulfonyl group, a sulfanyl group, a silyl group, a carbamoyl group, an aryl group, a heterocyclo group, an alkenyl group, an alkynyl group, a nitro group, a formyl group, a nitroso group, a formyloxy group, an isocyano group, a cyanate group, an isocyanate group, a thiocyanate group, an isothiocyanate group or cyano group; or an adjacent set of R₁ to R₄ are fused to form a substituted or unsubstituted ring.
It should be noted that: when R₁ and R₄ are the phenyl group, X and Y are not the alkyl group and phenyl group; when R₁ and R₄ are a thienyl group, conditions of X and Y being a monovalent hydrocarbon group, R₂ and R₃ being the alkyl group, the aryl group or the alkenyl group and R₂ and R₃ being aliphatic groups bonded to form a ring are not satisfied at the same time; when R₁ and R₄ are the silyl group, R₂, R₃, X and Y each are not the monovalent hydrocarbon group having 1 to 6 carbon atomsor the hydrogen atom; and when benzene rings are fused at R₁ and R₂, X and Y are not the alkyl group and the phenyl group.

A borane derivative shown by the formula below is also preferable as a material of the electron injecting (transporting) layer.

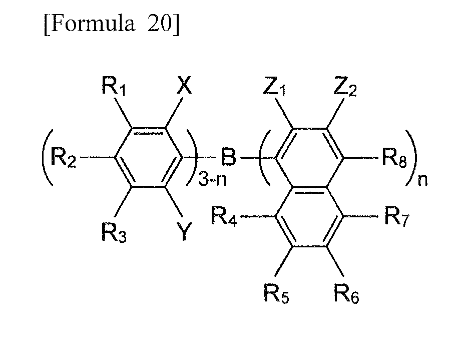

(In the formula above, R₁ to R₈ and Z₂ are each represent a hydrogen atom, a saturated or unsaturated hydrocarbon group, an aromatic group, a heterocyclo group, a substituted amino group, a substituted boryl group, an alkoxy group or an aryloxy group; X, Y and Z₁ are each represent a saturated or unsaturated hydrocarbon group, an aromatic group, a heterocyclo group, a substituted amino group, an alkoxy group or an aryloxy group; substituent groups of Z₁ and Z₂ may be bonded to form a fused ring; and n represents an integer of 1 to 3, where when n is equal to or larger than two, a plurality of Z₁ may be different from each other and a plurality of Z₂ may be different from each other.
However a condition in which n is 1, X, Y and R₂ are the methyl group and R₈ is the hydrogen atom or the substituted boryl group and a condition in which n is 3 and Z₁ is the methyl group are excluded.)

A gallium complex shown by the formula below is also preferable as a material of the electron injecting (transporting) layer.

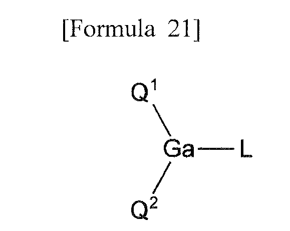

In this formula above, Q¹ and Q² each represent a ligand shown by the formula below. L represents a ligand which may be a halogen atom; a substituted or unsubstituted alkyl group; a substituted or unsubstituted cycloalkyl group; a substituted or unsubstituted aryl group; a substituted or unsubstituted heterocyclic group; those represented by -OR¹ (R¹ representing a halogen atom, a substituted or unsubstituted alkyl group, a substituted or unsubstituted cycloalkyl group, a substituted or unsubstituted aryl group or a substituted or unsubstituted heterocyclic group); or those represented by -O-Ga-Q³(Q⁴) (Q³ and Q⁴ being the same as Q¹ and Q²)

In the formula. Q¹ to Q⁴ each represents a residue shown by the formula below, which may be exemplified by, but not limited to, a quinoline residue such as 8-hydroxyquinoline and 2-methyl-8-hydroxyquinoline.

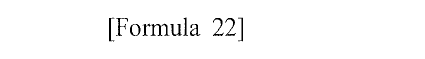

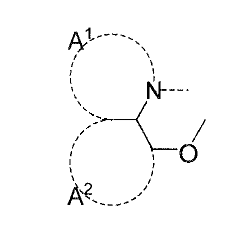

Rings A¹ and Ring A² are bonded to each other, Rings A¹ and A² being substituted or unsubstituted aryl rings bonded to each other or a heterocyclic structure.

The metal complex shown above exhibits a strong property as an n-type semiconductor and has a large electron injecting capability. In addition, formation energy required when forming the complex is low, so that bonding between the metal and the ligand in the formed metal complex becomes strong, thus exhibiting a large fluorescence quantum efficiency as an emitting material.

Concrete examples of the substituent groups of Ring A¹ and Ring A² that form the ligands in the formula above may include: halogen atoms of chlorine, bromine, iodine and fluorine; substituted or unsubstituted alkyl groups such as a methyl group, an ethyl group, a propyl group, a butyl group, a sec-butyl group, a tert-butyl group, a pentyl group, a hexyl group, a heptyl group, an octyl group, a stearyl group and a trichloromethyl group; substituted or unsubstituted aryl groups such as a phenyl group, a naphthyl group a 3-methylphenyl group, a 3-methoxyphenyl group, a 3-fluorophenyl group, a 3-trichloromethylphenyl group, a 3-trifluoromethylphenyl group and a 3-nitrophenyl group; substituted or unsubstituted alkoxy groups such as a methoxy group, a n-butoxy group, a tert-butoxy group, a trichloromethoxy group, a trifluoroethoxy group, a pentafluoropropoxy group, a 2,2,3,3-tetrafluoropropoxy group, a 1,1,1,3,3,3-hexafluoro-2-propoxy group and a 6-(perfluorohexyl)hexyloxy group; substituted or unsubstituted aryloxy groups such as a phenoxy group, a p-nitrophenoxy group, a p-tert-butylphenoxy group, a 3-fluorophenoxy group, a pentafluorophenyl group and a 3-trifluoromethylphenoxy group; substituted or unsubstituted alkylthio groups such as a methylthio group, an ethylthio group, a tert-butylthio group, a hexylthio group, an octylthio group and a trifluoromethylthio group; substituted or unsubstituted arylthio groups such as a phenylthio group, a p-nitrophenylthio group, a p-tert-butylphenylthio group, a 3-fluorophenylthio group, a pentafluorophenylthio group and a 3-trifluoromethylphenylthio group; mono- or disubstituted amino groups such as a cyano group, a nitro group, an amino group, a methylamino group, a dimethylamino group, an ethylamino group, a diethylamino group, a dipropylamino group, a dibutylamino group and a diphenylamino group; acylamino groups such as a bis(acetoxymethyl) amino group, a bis(acetoxyethyl) amino group, a bis (acetoxypropyl) amino group and a bis(acetoxybutyl) amino group; a hydroxyl group; a siloxy group; an acyl group; carbamoyl groups such as a methylcarbamoyl group, a dimethylcarbamoyl group, an ethylcarbamoyl group, a diethylcarbamoyl group, ***a*** propylcarbamoyl group, a butylcarbamoyl group, and a phenylcarbamoyl group; carboxylic acid groups; sulfonic acid groups; imide groups; cycloalkyl groups such as a cyclopentane group and a cyclohexyl group; aryl groups such as a phenyl group, a naphthyl group, a biphenyl group, an anthranil group, a phenanthryl group, a fluorenyl group and a pyrenyl group; and heterocyclic groups such as a pyridinyl group a pyrazinyl group, a pyrimidinyl group, a pyridazinyl group, a triazinyl group, an indolinyl group, a quinolinyl group, an acridinyl group, a pyrrolidinyl group, a dioxanyl group, a piperidinyl group, a morpholidinyl group, a piperazinyl group, a carbazolyl group, a furanyl group, a thiophenyl group, an oxazolyl group, an oxadiazolyl group, a benzoxazolyl group, a thiazolyl group, a thiadiazolyl group, a benzothiazolyl group, a triazolyl group, an imidazolyl group and a benzoimidazolyl group. In addition, the substituent groups listed above may be bonded to each other to form a 6-membered aryl ring or a heterocycle.

As a preferred embodiment of the organic EL device, there is known a device containing a reductive dopant at a boundary between a region transporting the electron or the cathode and an organic layer. Here, the reductive dopant is defined as a substance capable of reducing an electron transporting compound. Thus, various substances having a certain level of reducibility can be used, preferable examples of which may be at least one substance selected from the group consisting of: alkali metal, alkali earth metal, rare earth metal, an oxide of the alkali metal, a halogenide of the alkali metal, an oxide of the alkali earth metal, a halogenide of the alkali earth metal, an oxide of the rare earth metal, a halogenide of the rare earth metal, an organic complex of the alkali metal, an organic complex of the alkali earth metal and an organic complex of the rare earth metal.

Specifically, more preferable reductive dopant may be those having the work function of 2.9 eV or lower, which may be exemplified by at least one alkali metal selected from the group consisting of Li (work function: 2.9 eV), Na (work function: 2.36 eV), K (work function: 2.28 eV), Rb (work function: 2.16 eV) and Cs (work function: 1.95 eV) or at least one alkali earth metal selected from the group consisting of Ca (work function: 2.9 eV), Sr (work function: 2.0 to 2.5 eV) and Ba (work function: 2.52 eV), and the substances having the work function of 2.9 eV or lower are particularly preferable. Among these, more preferable reductive dopant is at least one alkali metal selected from the group consisting of K, Rb and Cs, in which Rb and Cs are even more preferable and Cs is most preferable. These alkali metals have particularly high reducibility, so that addition of a relatively small amount of these alkali metals to an electron injecting region can enhance luminescence intensity and lifecycle of the organic EL device. In addition, as the reductive dopant having the work function of 2.9 eV or lower, a combination of two or more of these alkali metals is also preferable, and a combination including Cs is particularly preferable, e.g., combinations of Cs an Na, Cs and K, Cs and Rb or Cs, Na and K. The combinations including Cs can effectively exert the reducibility, so that by adding such reductive dopant to the electron injecting region, the luminescence intensity and the lifecycle of the organic EL device can be enhanced.

An electron injecting layer formed from an insulator or a semiconductor may be provided between the cathode and the organic layer. With the arrangement, leak of electric current can be effectively prevented and the electron injecting capability can be enhanced. For the semiconductor, it is preferable to use at least one metal compound selected from the group consisting of an alkali metal chalcogenide, an alkaline earth metal chalcogenide, a halogenide of alkali metal and a halogenide of alkali earth metal. By forming the electron injecting layer from the alkali metal chalcogenide or the like, the electron injecting capability can further be enhanced, which is preferable. Specifically, preferable examples of the alkali metal chalcogenide may include Li₂O, K₂O, Na₂S, Na₂Se and Na₂O, while preferable example of the alkaline earth metal chalcogenide may include CaO, BaO, SrO, BeO, BaS and CaSe. Preferable examples of the halogenide of the alkali metal may include LiF, NaF, KF, LiCl, KCl and NaCl. Preferable examples of the halogenide of the alkali earth metal may include fluorides such as CaF₂, BaF₂, SrF₂, MgF₂ and BeF₂ and halogenides other than the fluoride.
Examples of the semiconductor for forming the electron transporting layer may include one type or a combination of two or more types of an oxide, a nitride or an oxidized nitride containing at least one element selected from the group consisting of Ba, Ca, Sr, Yb, Al, Ga, In, Li, Na, Cd, Mg, Si, Ta, Sb and Zn. An inorganic compound for forming the electron transporting layer is preferably a microcrystalline or amorphous semiconductor film. When the electron transporting layer is formed of such semiconductor film, more uniform thin film can be formed, thereby reducing pixel defects such as a dark spot. Examples of such inorganic compound may include the above-described alkali metal chalcogenide, alkali earth metal chalcogenide, halogenide of the alkali metal and halogenide of the alkali earth metal.

### [7] Cathode

In the cathode, metals, alloys, electrically conductive compounds and mixtures of the above, which each have a small work function (4 eV or lower), are used as an electrode material, in order to inject the electron to the electron injecting/transporting layer or the emitting layer. Concrete examples of the electrode material may include sodium, a sodium-potassium alloy, magnesium, lithium, a magnesium-silver alloy, aluminium/aluminium oxide, an aluminium-lithium alloy, indium and rare earth metal.
The cathode may be made by forming a thin film from these electrode substances by the vapor deposition and sputtering.
When emission from the emitting layer is taken out from the cathode, the cathode preferably has a transmittance of higher than 10% for the emission.
The sheet resistance as the cathodes is preferably several hundreds Ω/ square or lower, and the thickness of the film is typically in the range from 10nm to 1 µm, preferably 50 nm to 200 nm.

### [8] Insulating Layer

Since the electrical field is applied to ultra thin films in the organic EL device, pixel defects resulted from leak or short circuit likely occur. In order to prevent such defects, it is preferable to interpose an insulating thin film layer between a pair of electrodes.
Examples of materials used for the insulating layer may include aluminum oxide, lithium fluoride, lithium oxide, cesium fluoride, cesium oxide, magnesium oxide, magnesium fluoride, calcium oxide, calcium fluoride, aluminium nitride, titanium oxide, silicon oxide, germanium oxide, silicon nitride, boron nitride, molybdenum oxide, ruthenium oxide and vanadium oxide.
Mixtures or laminates of the above may also be used.

### [9] Manufacturing Method of Organic EL Device

The organic EL device can be manufactured by forming the anode, the emitting layer, the hole injecting layer (as needed), the electron injecting layer (as needed) and the cathode using the materials and formation methods mentioned above as examples. Also, the organic EL device can be manufactured by forming the above elements in the inverse order of the above, namely from the cathode to the anode.

The following is an example of a manufacturing method of the organic EL device in which the anode, the hole injecting layer, the emitting layer, the electron injecting layer and the cathode are sequentially formed on the light-transmissive substrate.

First, a thin film of the anode material is formed on a proper light-transmissive substrate by the vapor deposition or the work function such that the thickness of the thin film is 1 µm or smaller, preferably in the range from 10 nm to 200 nm.
Then, the hole injecting layer is formed on the anode. The hole injecting layer can be formed by the vacuum deposition, the spin coating, the casting method, the LB method or the like. The thickness of the hole injecting layer is properly selected from the range from 5 nm to 5 µm.

Then, the emitting layer is formed on the hole injecting layer by forming a thin film from an organic luminescent material by a dry process represented by the vacuum deposition or a wet process such as the spin coating and the casting method. However, the wet process is more preferable in terms of size increase in screen, reduction of cost and simplification of manufacturing process.

Then, the electron injecting layer is formed on the emitting layer. The vacuum deposition can be exemplified as a method for forming the electron injecting layer.

Lastly, the cathode is deposited, and the organic EL device can be obtained. The cathode is formed from metal by the vapor deposition, the sputtering or the like. In order to protect the organic layers deposited under the cathode from being damaged, the vacuum deposition is preferable.

The methods for forming each of the layers of the organic EL device are not particularly limited.
Conventional methods such as the vacuum deposition and the spin coating can be employed for forming the organic film layers. Specifically, the organic film layers may be formed by conventional coating methods such as the vacuum deposition, molecular beam epitaxy (MBE method) and coating methods using a solution such as the spin coating, the casting method, bar coating, roll coating and ink jet printing.
Although the thickness of each organic layer of the organic EL device is not particularly limited, the thickness is generally preferably in the range from several nanometers to 1 µm, since too small thickness likely cause defects such as a pin hole while too large thickness requires high voltage to be applied and lowers efficiency.
In a state where a direct current is applied to the organic EL device, when a voltage of 5 to 40 V is applied with the anode having the positive polarity and the cathode having the negative polarity, the luminescence can be observed. When the voltage is applied with the inversed polarity, the current is not applied, so that the luminescence is not generated. In a state where an alternating current is applied, the uniform luminescence can be observed only when the anode has the positive polarity and the cathode has the negative polarity. A waveform of the alternating current to be applied can be selected arbitrarily.

### (Example 6)

An example of how to manufacture the organic EL device using an ink (organic EL material-containing solution) will be described.
A glass substrate (size: 25 mm x 75 mm x 1.1 mm thick) having an ITO transparent electrode (manufactured by GEOMATEC Co., Ltd.) is ultrasonic-cleaned in isopropyl alcohol for five minutes, and then UV/ozone-cleaned for 30 minutes. Polyethylene-dioxy-thiophene/ polystyrene sulphonic acid (PEDOT/PSS) to be used for the hole injecting layer was deposited on the substrate by the spin coating to form a film having a thickness of 100 nm. Then, a toluene solution (0.6 wt%) of Polymer 1 shown below (Mw: 145000) was deposited by the spin coating to form a film having a thickness of 20 nm, which was dried at 170°C for 30 minutes. A toluene solution containing 1 wt% of Compound H2 and Compound A (Compound H2 : Compound A = 20/1 (wt/wt)) was deposited by the spin coating to form a film of the emitting layer. The thickness was 50 nm at this time. On the emitting layer, Compound B was deposited to form a film having a thickness of 30 nm. Li (Li source: manufactured by SAES Getters) as the reductive dopant and Alq are co-deposited to form Alq:Li film as the electron injecting layer (cathode). Metal (Al) was vapor-deposited on the Alq:Li film to form a metal cathode to complete the organic EL device. The device emitted a red light, had a uniform light emitting surface and had luminous efficiency of 5.5 cd/A.

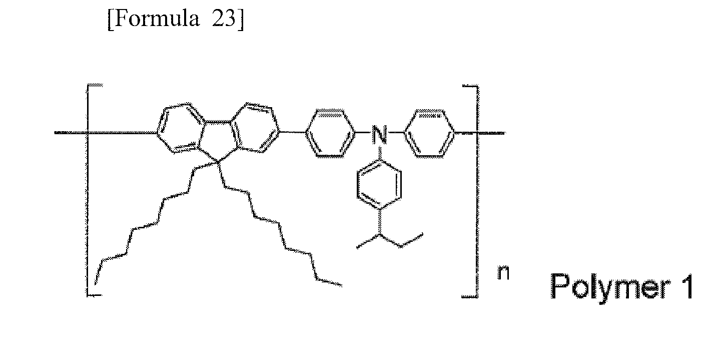

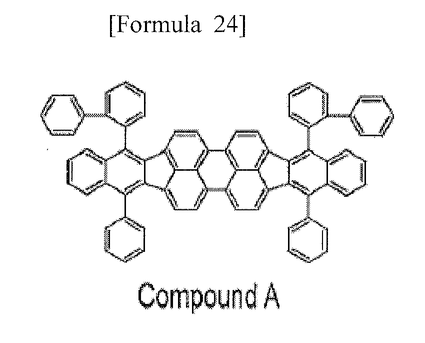

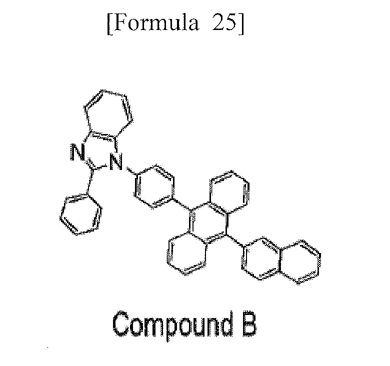

### (Comparison 6)

An organic EL device was formed similarly to Example 6 except that Compound h1 shown below having solubility in toluene of 1.4 wt% was used instead of Compound H2. Although the device emitted a red light, its luminous efficiency was 0.2 cd/A.

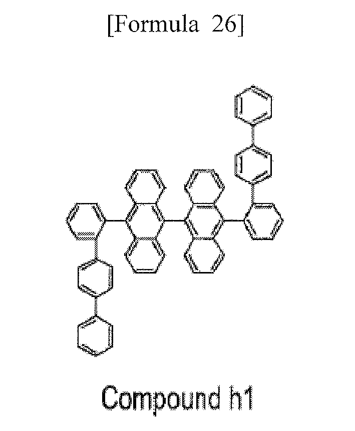

Although an anthracene compound (h1) having a solubility usable in film forming by the coating method was employed as a host compound for the red-light emission, the efficiency was low. From the result above, it was verified that the naphthacene compound had an excellent performance as the host compound for the red-light emission. In other words, the naphthacene compound obtained from the experiments above has both the solubility suitable for the coating process and the performance as the organic EL device.

### INDUSTRIAL APPLICABILITY

This invention can be used in manufacturing an organic electroluminescent display.

## Claims

1. An organic EL material-containing solution, comprising:
an organic EL material; and
a solvent, wherein
the organic EL material contains a host and a dopant,
the solvent contains a solvent selected from the group consisting of an aromatic solvent, a halogen type solvent and an ether type solvent, and
the host is shown by General Formula (1) below, the host having a solubility in the solvent of 0.5 wt% or higher, 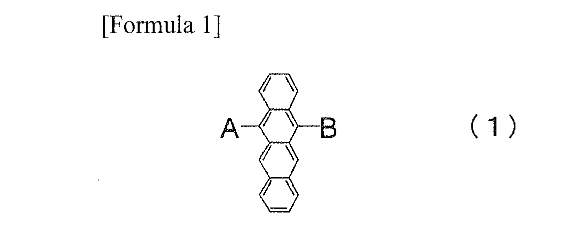 (in Formula (1) above, A and B each representing a substituted or unsubstituted aromatic group having 6 to 20 carbon atoms or a substituted or unsubstituted fused aromatic group having 10 to 20 carbon atoms, where A and B may be the same or different from each other, at least one of A and B having a structure shown by General Formula (2) below, 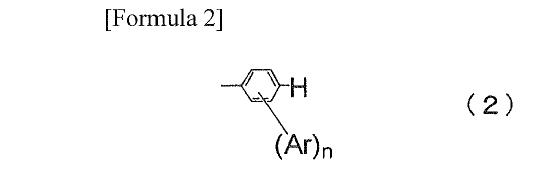 in the formula above, Ar representing a substituted or unsubstituted aromatic group having 6 to 20 carbon atoms or a substituted or unsubstituted fused aromatic group having 10 to 20 carbon atoms, n representing an integer of 0 to 4).

2. The organic EL material-containing solution according to claim 1, wherein, in Formula (2) above, n represents an integer of 0 to 2.

3. The organic EL material-containing solution according to claim 1 or 2, wherein , in Formula (2) above, Ar represents a substituted or unsubstituted aromatic group having 6 to 20 carbon atoms.

4. The organic EL material-containing solution according to any one of claims 1 to 3, wherein
the solvent is selected from the group consisting of the aromatic solvent, the halogen type solvent and the ether type solvent,
the host is shown by Formula (1) above, the host having a solubility in the solvent of 2 wt% or higher, and
a viscosity control reagent is added to the solvent, the viscosity control reagent being selected from the group consisting of an alcohol type solution, a ketone type solution, a paraffin type solution and an alkyl-substituted aromatic solution having 4 or more carbon atoms, in Formula (2) above, Ar being selected from the group consisting of phenyl, naphthyl and biphenyl.

5. The organic EL material-containing solution according to claim 4, wherein , in Formula (2) above, Ar represents a substituted or unsubstituted phenyl group.

6. The organic EL material-containing solution according to claim 4 or 5, wherein the solvent is the aromatic solvent, and
the viscosity control reagent is selected from the group consisting of the alcohol type solution and the alkyl-substituted aromatic solution having 4 or more carbon atoms.

7. The organic EL material-containing solution according to any one of claims 1 to 6, wherein the dopant is an indenoperylene derivative shown by Formula (3) below, 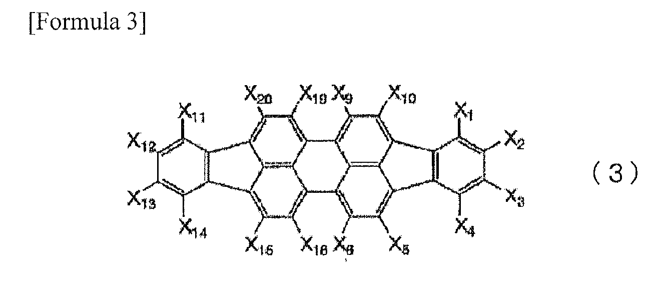 (In formula (3), X₁ to X₆, X₉, X₁₀, X₁₁ to X₁₆, X₁₉ and X₂₀ each represent a hydrogen, a halogen, an alkyl group, an alkoxy group, an alkylthio group, an alkenyl group, an alkenyloxy group, an alkenylthio group, an aromatic ring-containing alkyl group, an aromatic ring-containing alkyloxy group, an aromatic ring-containing alkylthio group, an aromatic ring group, an aromatic heterocyclic group, an aryloxy group, an arylthio group, an arylalkenyl group, an alkenylaryl group, an amino group, a carbazolyl group, a cyano group, a hydroxyl group, -COOR^{1'} (R^{1'} representing a hydrogen, an alkyl group, an alkenyl group, an aromatic ring-containing alkyl group or an aromatic ring group), -COR^{2'} (R^{2'} representing a hydrogen, an alkyl group, an alkenyl group, an aromatic ring-containing alkyl group, an aromatic ring group or an amino group) or -OCOR^{3'} (R^{3'} representing an alkyl group, alkenyl group, an aromatic ring-containing alkyl group or an aromatic ring group), adjacent groups of X₁ to X₆, X₉, X₁₀, X₁₁ to X₁₆, X₁₉ and X₂₀ may be bonded to each other or bonded to a substituted carbon atom to form a ring, and at least one of X₁ to X₆, X₉, X₁₀, X₁₁ to X₁₆, X₁₉ and X₂₀ is not the hydrogen).

8. The organic EL material-containing solution according to claim 7, wherein the indenoperylene derivative is shown by General Formula (4) below, 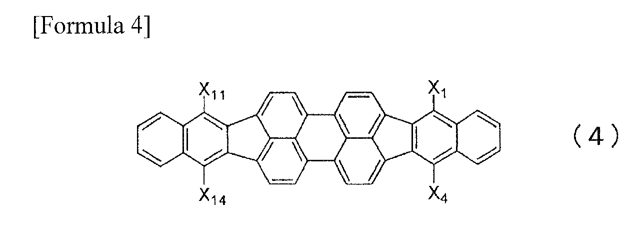 (in formula (4), X_{1,} X₄, X₁₁ and X₁₄ each being an aromatic ring group).

9. A method of forming thin film(s) of an organic EL material, comprising:
dropping the organic EL material-containing solution according to any one of claims 1 to 8 on a formation area; and
forming film(s) of the organic EL material by evaporating the solvent in the organic EL material-containing solution dropped in the step of dropping.

10. A thin film(s) of an organic EL material formed by the method of forming thin film(s) of an organic EL material according to claim 9.

11. An organic EL device, comprising: the thin film(s) of an organic EL material according to claim 10.
